(19)

Europäisches
Patentamt
European
Patent Office
Office européen
des brevets

(11) **EP 2 816 331 A1**

(12) **EUROPEAN PATENT APPLICATION**

(43) Date of publication:
**24.12.2014 Bulletin 2014/52**

(51) Int Cl.:
*G01G 19/50* (2006.01)    *A61B 5/053* (2006.01)

(21) Application number: **14171730.6**

(22) Date of filing: **10.06.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **19.06.2013 JP 2013128113**

(71) Applicant: **Tanita Corporation
Tokyo 174-8630 (JP)**

(72) Inventors:
• **Nakamura, Eiji
  Itabashi-ku
  Tokyo 174-8630 (JP)**

• **Honda, Daisuke
  Itabashi-ku
  Tokyo 174-8630 (JP)**

(74) Representative: **Haley, Stephen
  Gill Jennings & Every LLP
  The Broadgate Tower
  20 Primrose Street
  London EC2A 2ES (GB)**

Remarks:
A request for correction of parts of the description,
claims and drawings has been filed pursuant to Rule
139 EPC. A decision on the request will be taken
during the proceedings before the Examining Division
(Guidelines for Examination in the EPO, A-V, 3.).

(54)    **Body composition measuring apparatus and body composition measurement system**

(57)    A body composition measuring apparatus for calculating one or multiple types of body composition data is provided with a CPU configured to perform the following processes. The CPU sets second information indicating physical characteristics of a human subject. The CPU measures the bioelectrical impedance of the human subject by a voltage measurement circuit. The CPU obtains at least one piece of first information from each of at least one recording medium having recorded thereon the at least one piece of the first information necessary for calculating body composition data indicating a particular type of body composition, and calculates body composition data indicating a type of body composition corresponding to each of the at least one piece of the first information based on the at least one piece of the first information obtained by the information obtainer, the second information, and the bioelectrical impedance.

EP 2 816 331 A1

**Description**

BACKGROUND OF THE INVENTION

Field of the Invention

The present invention relates to a body composition measuring apparatus for measuring a body composition of a living body and to a body composition measurement system.

Description of Related Art

Conventionally, there is known a body composition measuring apparatus that has a function of measuring body composition data indicating the body composition of a human subject based on a bioelectric impedance measurement method (Bioelectrical Impedance Analysis: BIA). Body composition data measureable by such a body composition measuring apparatus includes various types of body composition data, for example, body fat percentage, lean body mass, visceral fat area, basic metabolic rate, bone mass, basic metabolism, etc.
In a body composition measuring apparatus having measurement functions of many such types of body composition data, a large number of types of body composition data are calculated as measurement results. Therefore, an operation of selecting a desired type from a large number of types of body composition data so that the desired data is displayed sometimes makes a user feel that it is troublesome.
To avoid such inconvenience, for example, in a living body measurement apparatus in Japanese Patent Application Laid-Open Publication No. 2007-117525 (hereinafter referred to as Patent Document 1), a key switch is provided corresponding to respective types of body composition data as a switch for making a desired body composition data be displayed with a simple operation.
A desired type of body composition data to be measured varies among users. Therefore, an extremely large number of measurement functions have to be provided in a body composition measuring apparatus if every function that could possibly be desired by multiple users is provided. In a case in which the body composition measuring apparatus is configured in this way, even if the technique disclosed in Patent Document 1 is used, the number of key switches will be extremely large, and the troublesomeness of operation still cannot be avoided. In particular, if many switches are provided for functions not desired by a user, the user tends to be annoyed by the excessive number of switches that the user does not even use.
On the other hand, if a body composition measuring apparatus is provided only with measurement functions for major (general) types of body composition data, such measurement functions do not always include all measurement functions desired by each user. Given the above situations, it is desired that a body composition measuring apparatus provided with measurement functions fitted to the needs of each user be provided.

Summary of the Invention

The present invention was made in consideration of the above situations, and has, as one of its objects, to provide a body composition measuring apparatus and a body composition measurement system for which measurement functions can be customized according to the needs of each user.
To solve the above problem, according to one aspect of the present invention, the body composition measuring apparatus for calculating at least one type of body composition data, the body composition measuring device has: an information obtainer that obtains at least one piece of first information from each of at least one recording medium having recorded thereon the at least one piece of the first information necessary for calculating body composition data indicating a particular type of body composition; an information setting unit that sets second information indicating physical characteristics of a human subject; a measurer that measures bioelectrical impedance of the human subject; and a calculator that calculates body composition data indicating a type of body composition corresponding to each of the at least one piece of the first information based on the at least one piece of the first information obtained by the information obtainer, the second information, and the bioelectrical impedance.
Thus configured, a body composition measuring apparatus with only the measurement functions of types of body composition data desired by a user can be provided. That is, a user only has to purchase a recording medium for adding a measurement function for a desired body composition data and to connect the medium to a body composition measuring apparatus being a platform provided with only minimum required measurement functions, whereby a body composition measuring apparatus with minimum required functions for the user can be provided. In the present invention, the human subject may be an object of a measurement, and at the same time, is a user of the body composition measuring apparatus. Alternatively, the human subject may be a person who is an object of a measurement when the body composition measuring apparatus is operated by another person who is a user of the body composition measuring apparatus.

Furthermore, in a case in which a new measurement function needs to be added after the purchase of the body composition measuring apparatus, measurement functions can be extended easily. Furthermore, because it is possible to configure a body composition measuring apparatus as a platform only provided with minimum measurement functions such as a weight measurement function, etc., it is possible to provide a body composition measuring apparatus that is simple and has excellent designed properties.

In a preferred embodiment, the first information may be a calculation program for calculating the body composition data by using the second information and the bioelectrical impedance, and the calculator, when the calculation program is obtained by the information obtainer, may execute the calculation program, thereby to calculate the body composition data by using the second information and the bioelectrical impedance.

Thus configured, the same effects as the above can be attained. Furthermore, a measurement function of a type of body composition data that was not expected at the time of selling the body composition measuring apparatus, and new types of body composition data that have become measurable due to the progress of technology, can be easily added to the body composition measuring apparatus, which the user has purchased.

In another preferred embodiment, the body composition measuring apparatus may additionally have a storage device having stored in advance multiple calculation programs, each for calculating the body composition data by using the second information and the bioelectrical impedance; each of the multiple calculation programs may be restricted from execution; the first information may be a restriction release key specifying a particular calculation program and instructing to release the restriction of the specified calculation program; and the calculator may read, from the storage device, the calculation program for which the restriction has been instructed to be released by the first information and release the restriction of the calculation program, and may execute the read calculation program by using the second information and the bioelectrical impedance, thereby to calculate the body composition data.

Thus configured, the same effects as the above can be attained. Furthermore, because a restriction release key is recorded in a recording medium, the required storage capacity of the recording medium can be reduced in comparison with a case in which the calculation program is recorded therein. Furthermore, although a restriction release key is transmitted from the recording medium to the body composition measuring apparatus, no calculation program is transmitted. Supposedly, if a calculation program were transmitted and if the calculation program were intercepted in the midst of transmission, the calculation program might be analyzed, or the calculation program might be diverted for use in a fraudulent device. However, because only the restriction release key is transmitted according to this preferred embodiment, the fraudulent acquisition, etc., of a calculation program that possibly takes place due to the transmission of the calculation program can be prevented.

According to another aspect of the present invention, a body composition measuring apparatus for calculating at least one type of body composition data, the body composition measuring device has: an information obtainer that obtains at least one piece of first information from each of at least one recording medium having recorded thereon at least one piece of the first information necessary for calculating body composition data indicating a particular type of body composition; an information setting unit that sets second information indicating physical characteristics of a human subject; a measurer that measures bioelectrical impedance of the human subject; a first storage device that stores the at least one piece of first information obtained from each recording medium by the information obtainer; a calculator that calculates body composition data indicating a type of body composition corresponding to each of the at least one piece of the first information based on the at least one piece of the first information, the second information, and the bioelectrical impedance; and a controller that reads, from the first storage device, at least one piece of first information from among the at least one piece of first information stored in the first storage device, for supply to the calculator.

Thus configured, a body composition measuring apparatus with only measurement functions of types of body composition data desired by a user can be provided. That is, a user only has to purchase a recording medium for adding a measurement function for a desired body composition data and connect the medium to a body composition measuring apparatus being a platform provided with only minimum required measurement functions, whereby the body composition measuring apparatus with minimum required functions for the user can be provided.

Furthermore, in a case in which a new measurement function needs to be added after the purchase of the body composition measuring apparatus, measurement functions can be extended easily. Furthermore, because it is possible to configure a body composition measuring apparatus as a platform provided with only minimum measurement functions such as a weight measurement function, etc., it is possible to provide a body composition measuring apparatus that is simple and has excellent designed properties.

Furthermore, because there is no need to connect a recording medium to the body composition measuring apparatus every time body composition data is measured, inconvenience of a user in actually measuring the body composition data can be avoided.

In a preferred embodiment, the first information may be a calculation program for calculating the body composition data by using the second information and the bioelectrical impedance; and the calculator, when the calculation program is supplied by the controller, may execute the calculation program, to calculate the body composition data by using the second information and the bioelectrical impedance.

**EP 2 816 331 A1**

Thus configured, the same effects as the above can be attained. Furthermore, a measurement function of a type of body composition data that was not expected at the time of selling the body composition measuring apparatus and a new type of body composition data that has become measurable due to the progress of technology can be easily added to the body composition measuring apparatus, which the user has purchased.

In another preferred embodiment, the body composition measuring apparatus may additionally have a second storage device having stored in advance multiple calculation programs, each for calculating the body composition data by using the second information and the bioelectrical impedance, each of the multiple calculation programs being restricted from execution; the first information may be a restriction release key specifying a particular calculation program and instructing to release the restriction of the specified calculation program; the controller may supply the calculator with the restriction release key, and may read, from the second storage device, the particular calculation program for supply to the calculator, the particular calculation program being specified by the restriction release key; and the calculator, when the restriction release key and the particular calculation program are supplied by the controller, may release the restriction of the particular calculation program and execute the read calculation program, thereby to calculate the body composition data by using the second information and the bioelectrical impedance.

Thus configured, the same effects as the above can be attained. Furthermore, because a restriction release key is recorded in a recording medium, the required storage capacity of the recording medium can be reduced in comparison with a case in which the calculation program is recorded therein. Furthermore, although a restriction release key is transmitted from the recording medium to the body composition measuring apparatus, a calculation program is not transmitted. Supposedly, if a calculation program were transmitted and if the calculation program were intercepted in the midst of transmission, the calculation program might be analyzed, or the calculation program might be diverted for use in a fraudulent device. However, because only the restriction release key is transmitted according to this preferred embodiment, the fraudulent acquisition, etc., of a calculation program that possibly takes place due to the transmission of the calculation program can be prevented.

According to still another aspect of the present invention, a body composition measuring apparatus has: a calculation program storage device that stores therein multiple calculation programs, each for calculating body composition data indicating body composition of a human subject, with each of the multiple calculation programs being restricted from execution; an information obtainer that obtains at least one piece of first information from each of at least one recording medium, the first information being a restriction release key specifying a particular calculation program and instructing to release the restriction of the particular calculation program; an information setting unit that sets second information indicating physical characteristics of the human subject; a measurer that measures bioelectrical impedance of the human subject; a controller that reads, from the calculation program storage device, the particular calculation program specified by the first information obtained by the information obtainer, and stores an identifier in association with the calculation program in the calculation program storage device, the identifier indicating that the restriction is to be released; and a calculator that executes the calculation program associated with the identifier, thereby to calculate the body composition data by using the second information and the bioelectrical impedance.

Thus configured, a body composition measuring apparatus only with measurement functions of types of body composition data desired by a user can be provided. That is, a user only has to purchase a recording medium for adding a measurement function for a desired body composition data and connect the medium to a body composition measuring apparatus being a platform provided with only minimum required measurement functions, whereby the body composition measuring apparatus with minimum required functions for the user can be provided.

Furthermore, in a case in which a new measurement function needs to be added after the purchase of the body composition measuring apparatus, measurement functions can be extended easily. Furthermore, because it is possible to configure a body composition measuring apparatus as a platform only provided with minimum measurement functions such as a weight measurement function, etc., it is possible to provide the body composition measuring apparatus that is simple and has excellent designed properties.

Furthermore, because there is no need to connect a recording medium to the body composition measuring apparatus every time body composition data is measured, inconvenience of a user in actually measuring the body composition data can be avoided. Moreover, supposedly, if a calculation program were transmitted and if the calculation program were intercepted in the midst of transmission, the calculation program might be analyzed, or the calculation program might be diverted for use in a fraudulent device. However, because only the restriction release key is transmitted according to this aspect of the present invention, the fraudulent acquisition, etc., of a calculation program that possibly takes place due to the transmission of the calculation program can be avoided.

In a preferred embodiment, the body composition measuring apparatus may additionally have a notifier that notifies the human subject of a type of body composition data corresponding to the at least one piece of first information when the body composition measuring apparatus changes to a state in which the at least one piece of first information can be obtained from the at least one recording medium.

Thus configured, the same effects can be attained as those of one of the above aspects and embodiments. Furthermore, because a user is able to easily understand a type of body composition data measurable at present, tasks

4

for having a desired type of body composition data measured are made easier.

In another preferred embodiment, the body composition measuring apparatus may additionally have multiple connectors, each connecting one of the at least one recording medium to the body composition measuring apparatus in a communicable manner.

Thus configured, the same effects can be attained as those of one of the above aspects and embodiments. Furthermore, a recording medium can be directly connected to the body composition measuring apparatus. That is, according to this embodiment, a simple body composition measurement system is actualized, requiring only the body composition measuring apparatus and the recording medium.

In still another preferred embodiment, the recording medium can be connected to (may be attached to or may be detached from) an information transmission device having and multiple connectors, each connecting one of the at least one recording medium to the information transmission device in a communicable manner, and (when the recording medium is attached to the information transmission device) an information transmitter that obtains the at least one piece of first information from each of the at least one recording medium connected to one of the multiple connectors and transmits wirelessly or by wire the obtained at least one piece of first information to the body composition apparatus, and the body composition measuring apparatus may additionally have an information receiver that receives the at least one piece of first information transmitted by the information transmission device.

Thus configured, the same effects can be attained as those of one of the above aspects and embodiments. Furthermore, because the recording medium does not have to be attached to the body composition measuring apparatus, the configuration of the body composition measuring apparatus can be simplified to a greater extent.

In a preferred embodiment, the recording medium may be provided in a communication terminal device that performs wireless or wire communication, and the information obtainer may receive the at least one piece of first information from the communication terminal device.

Thus configured, the same effects can be attained as those of one of the above aspects and embodiments. Furthermore, the body composition measuring apparatus can be made work in cooperation with a communication terminal device such as a smartphone, whereby a calculation program or a restriction release key can be obtained via the communication terminal device.

In another preferred embodiment, the body composition measuring apparatus may additionally have: a body composition data transmitter that transmits the body composition data to an activity meter that generates activity amount data indicating the amount of activity of the human subject; an activity amount data receiver that receives the activity amount data generated by the activity meter; and a data manager that stores the body composition data and the activity amount data in association with each other.

Thus configured, the same effects can be attained as those of one of the above aspects and embodiments. Furthermore, weight data and body composition data can be managed in association with the activity amount data generated by the activity meter.

Therefore, a user is able to easily understand timewise changes of the weight data, the body composition data, and the activity amount data, and the correlation between these data can be easily recognized. Furthermore, the activity amount data calculated by the activity meter reflects the latest body composition data calculated by the body composition measuring apparatus, and the accuracy in the activity amount data is enhanced.

According to one aspect of the present invention, a body composition measuring apparatus for calculating body composition data indicating body composition of a human subject has: an information setting unit that sets second information indicating physical characteristics of the human subject; a measurer that measures bioelectrical impedance of the human subject; a storage device that stores therein multiple calculation programs, each for calculating a particular type of the body composition data based on the physical characteristics information and the bioelectrical impedance, each of the calculation programs being restricted from execution and having embedded therein an unlocking process of releasing the restriction by a restriction release key for releasing the restriction; a restriction release key obtainer that obtains the restriction release key from a recording medium in which the restriction release key for at least one of the multiple calculation programs is stored; and a calculator that executes the calculation program for which the restriction has been released by the restriction release key, thereby to calculate the body composition data based on the physical characteristics information and the bioelectrical impedance.

Thus configured, a user merely has to select and purchase a recording medium in which a restriction release key for enabling execution of a measurement function of a desired type of body composition data and connect the recording medium to the body composition measuring apparatus, whereby the body composition measuring apparatus provided with functions desired by the human subject and the user can be actualized. Because a restriction release key is recorded in the recording medium, the required storage capacity of the recording medium can be reduced in comparison with a case in which the calculation program is recorded therein. Furthermore, although a restriction release key is transmitted from the recording medium to the body composition measuring apparatus, a calculation program is not transmitted. Supposedly, if a calculation program were transmitted and if the calculation program were intercepted in the midst of transmission, the calculation program might be analyzed, or the calculation program might be diverted for use in a fraudulent device. However, in this aspect of the present invention, because only the restriction release key is transmitted, security against the fraudulent use of a calculation program can be enhanced.

According to another aspect of the present invention, a body composition measurement system has the body composition measuring apparatus of any one of the above aspects and embodiments and a recording medium having recorded thereon the first information being information necessary for calculation of body composition data.

Thus configured, a body composition measuring system with only measurement functions of types of body composition data desired by a user can be provided. That is, a user merely has to purchase a recording medium for adding a measurement function for a desired type of body composition data and connect the medium to a body composition measuring apparatus being a platform provided with only minimum required measurement functions, whereby a body composition measuring system with minimum required functions for the user can be provided.

Furthermore, in a case in which a new measurement function needs to be added after the purchase of the body composition measuring apparatus, measurement functions can be extended easily. A body composition measuring apparatus is a platform provided with only minimum measurement functions such as a weight measurement function, etc., and therefore, it is possible to provide a body composition measuring apparatus that is simple and has superior designed properties.

BRIEF DESCRIPTION OF THE DRAWINGS

Fig. 1 is a perspective view showing an exterior configuration of a body composition measuring apparatus according to a first embodiment of the present invention.

Fig. 2A is a block diagram showing a system configuration example of the body composition measuring system according to the first embodiment of the present invention.

Fig. 2B is a functional block diagram of the body composition measuring system according to the first embodiment of the present invention.

Fig. 3 is a flowchart showing a flow of a measurement process of body composition data performed by the body composition measuring apparatus according to the first embodiment of the present invention.

Fig. 4 is a flowchart showing a flow of a measurement process of body composition data performed by a body composition measuring apparatus according to a second embodiment of the present invention.

Fig. 5 is a diagram showing a configuration example of an information transmission device for transmitting information recorded in a function adding device to a body composition measuring apparatus according to a third embodiment of the present invention.

Fig. 6 is a block diagram showing a system configuration example of the information transmission device for transmitting information recorded in a function adding device to a body composition measuring apparatus according to a third embodiment of the present invention.

DETAILED DESCRIPTION OF PREFERRED EMBODIMENTS

In the following, preferred embodiments of the present invention will be described in detail with reference to the attached drawings. However, in each of the drawings, the size and scale of each element are appropriately changed from the actual size and scale. Furthermore, because the following embodiments are preferred concrete examples of the present invention, various features have been added that are technically suitable. However, the scope of the present invention is not limited to those described in the following description unless otherwise stated.

First Embodiment

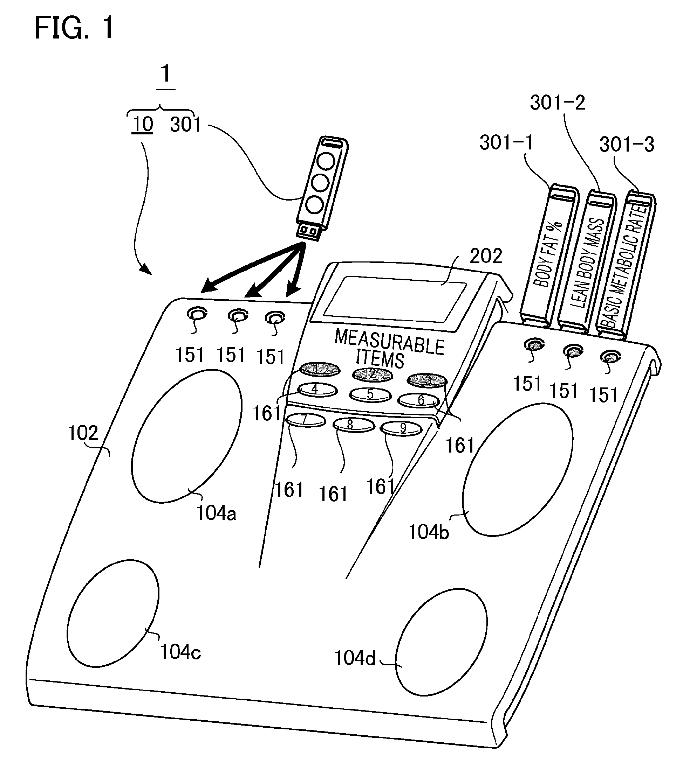

Fig. 1 is a perspective view showing an exterior configuration of a body composition measurement system 1 and a body composition measuring apparatus 10 according to a first embodiment of the present invention. The body composition measurement system 1 has a body composition measuring apparatus 10 with a weight measurement function and a function adding device 301 for adding, to the body composition measuring apparatus 10, measurement functions for various types of body composition data. In the present embodiment, the function adding device 301 is a recording medium in which a calculation program (first information) for calculating a particular body composition data is stored.

As shown in the figure, the top surface of the body composition measuring apparatus 10 is provided with a platform 102 that a human subject (not shown) steps on while measurements are taken. The platform 102 is provided with current supplying electrode 104a,104b, voltage measuring electrode 104c,104d, state display unit 151, function display unit 161, and display unit 202.

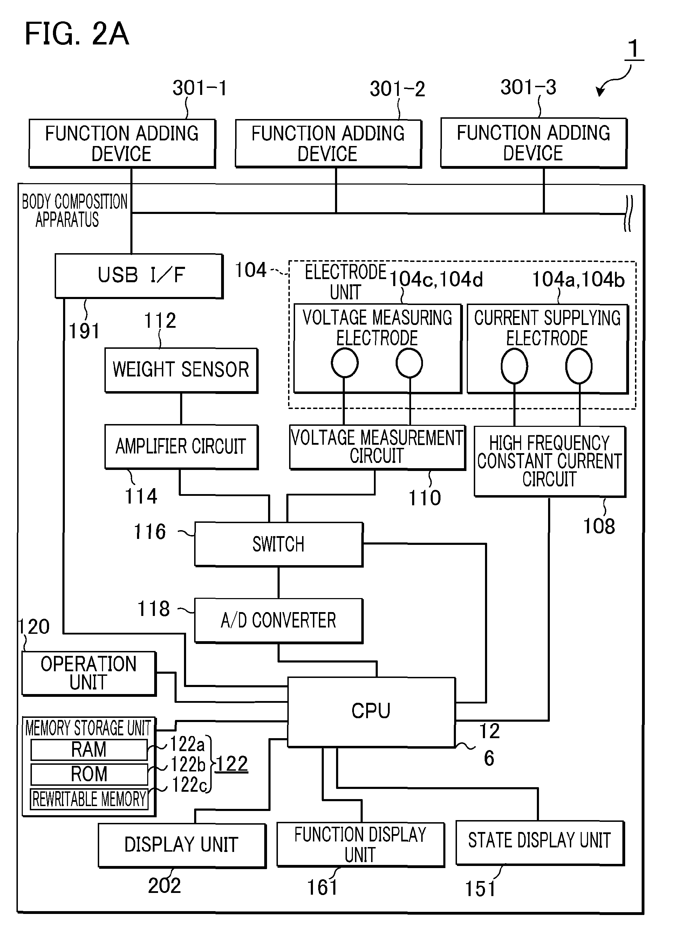

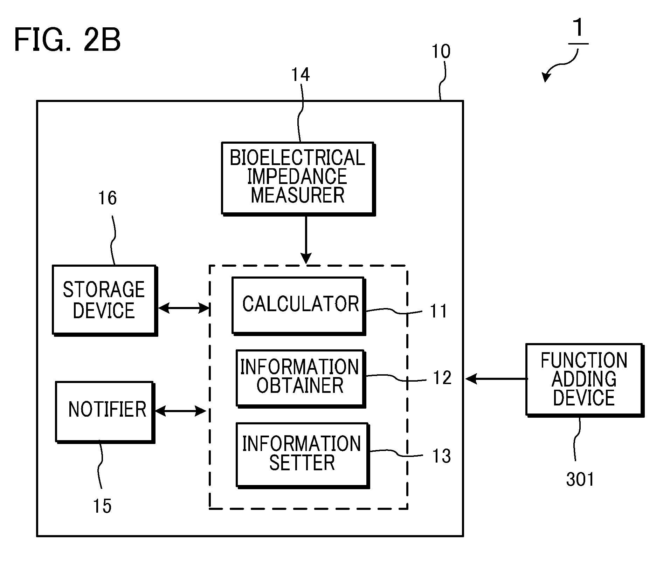

Fig. 2A is block diagram showing an example of a system configuration of the body composition measuring system 1 according to the first embodiment. Fig. 2B is a functional block diagram of the body composition measuring apparatus 10. As shown in Fig. 2A, the body composition measuring apparatus 10 has a CPU 126, an electrode unit 104, a high frequency constant current circuit 108, a voltage measurement circuit 110, a weight sensor 112, an amplifier circuit 114, a switch 116, an A/D converter 118, an operation unit 120, a memory storage unit 122, the state display unit 151, the function display unit 161, the display unit 202, and an interface 191.

As shown in Fig. 1 and 2A, the interfaces 191 accommodate (connect) the function adding devices 301 on the side portion of the upper side of the figure of the body composition measuring apparatus 1 at positions corresponding to respective state display units 151.

The CPU (Central Processing Unit) 126 is a controller that controls each unit of the body composition measuring apparatus 10, and executes various processes, as described later. The CPU 126 reads various computer programs stored in the storage device 122 to execute instruction codes of respective computer programs, including a computer program for a body composition measurement process according to the present embodiment, whereby various functions of the body composition measuring apparatus 10 are actualized. Furthermore, the CPU 126 executes a calculation program recorded in the function adding device 301, thereby calculating body composition data.

The memory storage unit 122 has a RAM (Random Access Memory) 122a, a ROM (Read Only Memory) 122b, and a rewritable memory 122c. The ROM 122b is a non-volatile memory having stored therein various computer programs, each for causing the CPU 126 to execute various processes and a calculation formula for calculating weight.

As shown in Fig. 2B, the body composition apparatus 10 includes, as functional elements, a calculator 11, an information obtainer 12, an information setting unit 13 that are implemented by the CPU 126 by executing the computer program for the body composition measurement process stored in the memory storage unit 122 as well as by executing the calculation program recorded in the functional adding device 301. The body composition apparatus 10 additionally has functional elements that are implemented by each unit of the apparatus under the control of the CPU 126. Specifically, the body composition apparatus 10 has, as such functional elements, a bioelectrical impedance measurer 14, a notifier 15, and a memory storage unit 16.

As shown in Figs. 1 and 2A, the current supplying electrode 104a,104b and the voltage measuring electrode 104c,104d are electrodes for measuring bioelectrical impedance between the feet of a human subject. The current supplying electrode 104a and the voltage measuring electrode 104c are electrodes on which a left foot of a human subject is placed. The current supplying electrode 104b and the voltage measuring electrode 104d are electrodes on which the right foot of a human subject is placed.

The high frequency constant current circuit 108 is a circuit for applying a small constant current of high frequency to the current supplying electrodes 104a and 104b. With the left foot of a human subject being placed on the current supplying electrode 104a and the voltage measuring electrode 104c, and the right foot of the human subject being placed on the current supplying electrode 104b and the voltage measuring electrode 104d, the high frequency constant current circuit 108 applies a small constant current of high frequency to the current supplying electrodes 104a and 104b. This constant current is applied to the soles of the human subject via the current supplying electrodes 104a and 104b.

The voltage measurement circuit 110 measures a potential difference generated between the voltage measuring electrodes 104c and 104d. That is, with a small constant current of high frequency being applied via the current supplying electrodes 104a and 104b to the soles of the human subject by the high frequency constant current circuit 108, the voltage measurement circuit 110 measures a potential difference between the voltage measuring electrodes 104c and 104d. Data showing the measured potential difference is converted to digital data by the A/D converter 118, and is supplied to the CPU 126 as bioelectrical impedance Z.

The weight sensor 112 measures the weight of a human subject. When the human subject steps on the platform 102, the weight sensor 112 measures and outputs a signal showing the weight of the human subject. The amplifier circuit 114 amplifies the signal output from the weight sensor 112. Specifically, the weight sensor 112 is provided with an elastic body and a strain gauge, and includes a load cell for measuring and outputting the change in voltage due to the strain of the elastic body. The signal output from the weight sensor 112 that has been amplified by the amplifier circuit 114 is converted to a digital signal by the A/D converter 118 and then is supplied to the CPU 126 as weight data W.

The switch 116, in response to a control signal from the CPU 126, switches a circuit connecting to the A/D converter 118 either to the voltage measurement circuit 110 or to the amplifier circuit 114.

The A/D converter 118 converts an analog signal into a digital signal, the analog signal being output from the voltage measurement circuit 110 and the amplifier circuit 114.

The state display unit 151 is a notifying means that is lit when the function adding device 301 is connected to the body composition measuring apparatus 10, thereby notifying a user of that effect. Specifically, the state display unit 151 is a lighting member such as an LED (Light Emitting Diode). When the function adding device 301 is connected to the body composition measuring apparatus 10, the state display unit 151 provided at a position corresponding to the interface 191 to which the function adding device 301 is connected is lit. Visually recognizing the lit or not lit condition of the state display unit 151, a user is able to readily determine whether the function adding device 301 is attached to the body composition measuring apparatus 10 in a communicable manner. In the present embodiment, the user is the object of measurements to be taken, i.e., a human subject. However, the user may be a person who operates the body composition measuring apparatus 10 to take measurements of another person who is a human subject.

The function display unit 161 is a notifying means for notifying a user of a function that can be added to the body composition measuring apparatus 10 and a function that has been added presently. Specifically, the function display unit 161 is a lighting member such as an LED, and in the present example, on the top of each function display unit 161 is a number, as shown in Fig. 1. When the function adding device 301 is connected to the body composition measuring apparatus 10, the function display unit 161 corresponding to a measurement function added by the function adding device 301 is lit. That is, the function display unit 161 serves as the notifier 15 that notifies when the body composition measuring apparatus 10 changes to a state in which the first information (calculation program in the present example) can be obtained from the recording medium (the function adding device 301 in the present example), that notifies the human subject of a type of body composition data corresponding to the first information.

One of the numbers from "1" to "9" written on each function display unit 161 corresponds to a particular function. In the present example, "1" indicates "body fat percentage measurement", "2" indicates "lean body mass measurement", and "3" indicates "basic metabolic rate measurement". Other numbers may be assigned body composition data such as "muscle mass", "bone mass", etc., or may be assigned a living body index data such as "visceral fat level", "body shape level", "metabolism age", etc.

Such configuration enables a user to easily know a function that can be added to the body composition measuring apparatus 10 and a function that has actually been added at present by viewing the function display unit 161.

To simplify the diagram, the numbers are written on each function display unit 161 for associating the function display unit 161 and each function, but instead of the numbers, different colors of illumination lights may be used for the respective functions display units 161, so that the colors of the lights and respective functions are associated. With such a configuration, a user simply has to visually recognize the color of the function display unit 161 that is lit, thereby to easily know the function that has actually been added at present.

Furthermore, the exterior of the function adding device 301 may be colored as corresponding to a color of the light associated with each function, whereby the function adding device 301 can be identified much more easily. Accordingly, when a user purchases the function adding device 301, for example, at a shop, etc., the user can easily and quickly identify and select the function adding device 301 corresponding to a desired measurement function.

The function adding device 301 is a device for adding a measurement function to the body composition measuring apparatus 10. In the present embodiment, the function adding device 301 is a recording medium (a USB memory medium in the present example) in which a calculation program for calculating particular body composition data is stored. As shown in Fig. 1, in the present example, a function adding device 301-1, a function adding device 301-2, and a function adding device 301-3 are connected to the body composition measuring apparatus 10 via the interface 191.

In other words, the body composition measuring apparatus 10 is configured so as to be communicable with a recording medium (the function adding device 301 in the present example) in which first information (a calculation program in the present example) necessary for calculating body composition data, and is a body composition measuring apparatus for calculating body composition data of a human subject. The function adding devices 301-1, 301-2, 301-3, ... are collectively called the function adding device 301 when description is given of a matter that is common to these function adding devices 301-1, 301-2, 301-3, etc.

The function adding device 301-1 is a recording medium having recorded therein a calculation program for calculating "body fat percentage", and is a function adding device for adding a body fat percentage measurement function to the body composition measuring apparatus 10. The function adding device 301-2 is a recording medium having recorded therein a calculation program for calculating "lean body mass", and is a function adding device for adding a lean body mass measurement function to the body composition measuring apparatus 10. The function adding device 301-3 is a recording medium having recorded therein a calculation program for calculating "basic metabolic rate", and is a function adding device for adding a basic metabolic rate measurement function to the body composition measuring apparatus 10. Thus, in the present example, each function adding device 301-1 to 301-3 has recorded therein one calculation program. However, each function adding device 301 may have recorded respectively therein multiple calculation programs.

Furthermore, each function adding device 301 has stored therein, together with a calculation program, information indicating a type of body composition data that can be measured by the calculation program, the information being recorded in association with the calculation program.

The interface 191 is an interface for connecting the function adding device 301 to the body composition measuring apparatus 10. In a case in which the function adding device 301 is configured as a USB memory medium as in the present example, the interface 191 is configured as a USB interface. In an example shown in Fig. 2A, via the interface 191, the function adding devices 301-1, 301-2, and 301-3 are connected to the body composition measuring apparatus 10 in a communicable manner. Thus, the interfaces 191 serve as multiple connectors that connect the recording mediums (the function adding devices 301 in the present example) to the body composition measuring apparatus in a communicable manner.

The display unit 202 is a display means such as an LCD (Liquid Crystal Display), etc. Displayed on the display unit 202 are measurement results, etc., of weight and various types of body composition data.

The operation unit 120 is a means for operating the body composition measuring apparatus 10.

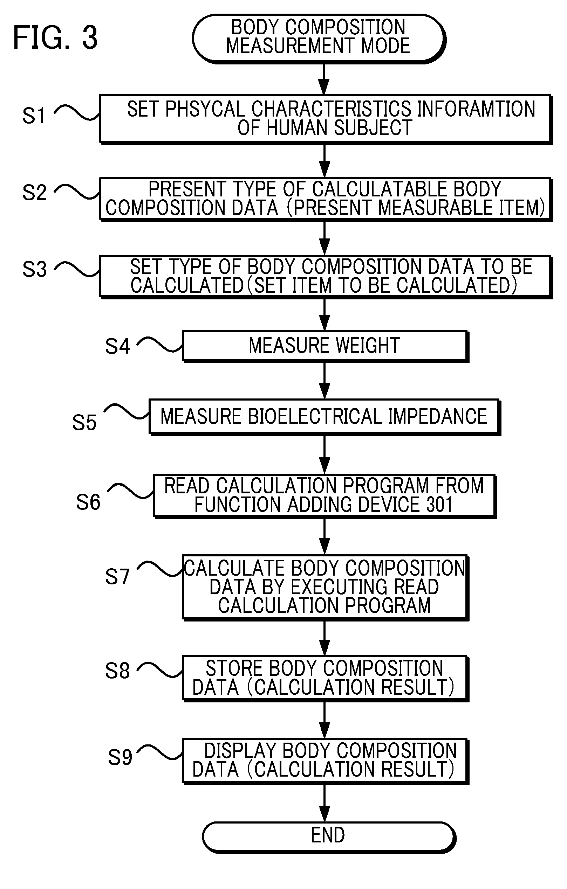

In the following, description is given of an example of a measurement process of body composition data performed at the body composition measuring apparatus 10 according to the first embodiment of the present invention. Fig. 3 is a flowchart showing a flow of the measurement process of body composition data performed by the body composition measuring apparatus.

First, when the body composition measuring apparatus 10 is turned on by an operation performed on the operation unit 120 by a human subject, the CPU 126 activates the body composition measuring apparatus 10 to cause an initial screen to be displayed on the display unit 202. The initial screen is, for example, an operation screen for setting modes, etc. When an operation to the operation unit 120 by the human subject is performed, the operation for selecting a "body composition measurement mode", the CPU 126 sets the body composition measuring apparatus 10 to the "body composition measurement mode". The "body composition measurement mode" is a mode for measuring body composition data of a human subject.

When the body composition measuring apparatus 10 is set to the body composition measurement mode, the CPU 126 first sets "physical characteristics information" about the body of the human subject (Step S1). The "physical characteristics information" is information indicating the physical characteristics of a human subject, the information being input to the body composition measuring apparatus 10 by an operation of the operation unit 120 by the human subject, and is information such as height, weight, age, sex, etc. The physical characteristics information, when it is input by a human subject, is stored in association with an identifier of the human subject in the rewritable memory 122c of the memory storage unit 122. In Step S1, the CPU 126 reads, from the rewritable memory 122c of the memory storage unit 122, the physical characteristics information of the human subject, to set it as physical characteristics information in the body composition measurement process. Thus, the CPU 126 serves as the information setting unit 13 that sets the physical characteristics information (second information) indicating the physical characteristics of a human subject.

Subsequently, the CPU 126 reads, from the function adding device 301 connected to the interface 191, "information indicating a type of body composition data" that can be calculated by a function added by the function adding device 301, to present to the user "a type of the body composition data" indicated by the information (Step S2). Specifically, the CPU 126 lights the function display unit 161 corresponding to a type of body composition data that can be calculated by a function provided by the function adding device 301 connected to the interface 191. A user simply has to check whether the state of the function display unit 161 is lit or not lit, thereby readily determining the type of body composition data that can be measured by the body composition measuring apparatus 10.

In the example shown in Figs. 1 and 2A, the function adding device 301-1 having recorded therein a calculation program for calculating "body fat percentage", the function adding device 301-2 having recorded therein a calculation program for calculating "lean body mass", and the function adding device 301-3 having recorded therein a calculation program for calculating "basic metabolic rate" are connected to the interfaces 191. Therefore, the state display units 151 corresponding to the interfaces are lit, and the function display unit 161 on which "1" associated with body fat percentage measurement is written, the function display unit 161 on which "2" associated with lean body mass measurement is written, and the function display unit 161 on which "3" associated with basic metabolic rate measurement is written are lit.

The human subject, having viewed the types of measurable body composition data ("measurable items") presented by the body composition measuring apparatus 10 in the process of Step S2, selects one or more characteristics the human subject wishes to have measured (desired type of body composition data) by an operation performed on the operation unit 120. The CPU 126 sets the type or types of body composition data selected by the human subject as an item or items to be measured (Step S3).

The human subject then steps on the platform 102 of the body composition measuring apparatus 10, with the left foot of the human subject being placed on the current supplying electrode 104a and the voltage measuring electrode 104c, and the right foot of human subject being placed on the current supplying electrode 104b and the voltage measuring electrode 104d. In this state, the CPU 126 measures the weight W of the human subject by the weight sensor 112 (Step S4), and measures bioelectrical impedance Z by the voltage measurement circuit 110 (Step S5). The weight W measured by the weight sensor 112 and the bioelectrical impedance Z measured by the voltage measurement circuit 110 are transmitted to the CPU 126 and are subsequently stored temporarily in the RAM 122a of the memory storage unit 122. Thus, the CPU 126, the weight sensor 122, and the voltage measurement circuit 110 serve as the bioelectrical impedance measurer 14 that measures bioelectrical impedance of a human subject.

The CPU 126 then reads a calculation program from each of the function adding devices 301 (Step S6). In other words, the CPU 126 serves as the information obtainer 12 that obtains at least one calculation program (at least one piece of first information) from each of at least one function adding device 301 (at least one recording medium) having recorded thereon the at least one calculation program necessary for calculating body composition data indicating a particular type of body composition.

Specifically, in the present example, the CPU 126 reads, from the function adding device 301-1 a calculation program for calculating the "body fat percentage", reads from the function adding device 301-2 a calculation program for calculating the "lean body mass", and reads from the function adding device 301-3 a calculation program for calculating the "basic metabolic rate".

Subsequently, the CPU 126 executes each of the calculation programs in Step S6, to calculate body composition data from the physical characteristics information and the bioelectrical impedance Z (Step S7). Thus, the CPU 126 serves as the calculator 11 that calculates body composition data indicating a type of body composition corresponding to each of the at least one calculation program (at least one piece of the first information) based on the at least one calculation program obtained by the information obtainer, the physical characteristics information (second information), and the bioelectrical impedance Z. In the following, the process in Step S7 will be described.

Body Fat Percentage Measurement

The CPU 126 executes the calculation program that is read from the function adding device 301-1, for obtaining body fat percentage %Fat, whereby the calculation of the following calculation formula (Formula 1) is executed, and whereby the body fat percentage is calculated (estimated):

$$\%\text{Fat} = \text{f1} * \text{Z} * \text{W} / \text{H}^2 - \text{f2} \ldots\ldots \text{(Formula 1)},$$

where f1 and f2 are coefficients and are values determined by the multiple regression analysis as appropriate. Furthermore, Z is the bioelectrical impedance of the human subject, W is the weight of the human subject, and H is the height of the human subject. The weight W and height H are the above-described physical characteristics information.

Lean Body Mass Measurement

The CPU 126 executes the calculation program that is read from the function adding device 301-2, for obtaining lean body mass FFM, whereby the calculation of the following calculation formula (Formula 2) is executed, and whereby the lean body mass is calculated (estimated):

$$\text{FFM} = \text{W} - \text{W} * \%\text{Fat} / 100 \ldots\ldots \text{(Formula 2)},$$

where %Fat is a value calculated by Formula 1.

Basic Metabolic Rate Measurement

The CPU 126 executes the calculation program that is read from the function adding device 301-3, for obtaining basic metabolic rate BMR, whereby the calculation of the following calculation formula (Formula 3) is executed, and whereby the basic metabolic rate is calculated (estimated):

$$\text{BMR} = \text{p1} * \text{FFM}^2 + \text{p2} * \text{FFM} + \text{p3} * (1 / \text{Age}) + \text{p4} \ldots\ldots \text{(Formula 3)},$$

where the coefficients p1 to p4 are values determined by multiple regression analysis as appropriate; Age is the age of the human subject; %Fat is a value calculated by Formula 1; and FFM is a value calculated by Formula 2.

When the calculation of body composition data in Step S7 is completed, the CPU 126 then causes the body composition data to be stored in the RAM 122a of the memory storage unit 122 (Step S8) and to be displayed on the display unit 202 (Step S9), and then ends the body composition measurement process. In the present example, in Step S9, values of the body fat percentage %Fat, the lean body mass FFM, and the basic metabolic rate BMR are displayed on the display unit 202. Not only the body composition data calculated in Step S7, but also the weight measured in Step S4, may be displayed on the display unit 202 together with the body composition data in Step S9.

As described above, according to the first embodiment of the present invention, it is possible to provide a body composition measuring apparatus and a body composition measurement system for which measurement functions can be customized in response to the needs of each user.

Specifically, a user simply has to purchase the function adding device 301 for a desired type of body composition data for connection to the body composition measuring apparatus 10, which is a platform, to configure the body composition measuring apparatus 10 having the minimum necessary functions for the user. Therefore, the body composition measuring apparatus 10 that is cost effective for a user and a manufacturer can be provided. Furthermore, in a case in which a need arises to extend the measurement function of the body composition measuring apparatus 10 after the purchase, it is possible to easily extend the measurement function.

Furthermore, because the body composition measuring apparatus 10 is a platform only provided with minimum necessary measurement functions, the body composition measuring apparatus 10 can be configured as one that is simple and has excellent designed properties.

Moreover, a measurement function of a type of body composition data that was not expected at the time of selling the body composition measuring apparatus 10 and a new type of body composition data that has become measurable due to the progress in technology can be easily added to the body composition measuring apparatus 10, which the user has purchased.

Body composition measuring apparatuses are often used in ordinary households. In a case in which a body composition measuring apparatus is used in an ordinary household, the body composition measuring apparatus records and manages weight data and body composition data. When such a body composition measuring apparatus is configured as the body composition measuring apparatus 10 or as the body composition measurement system 1 according to the first embodiment of the present invention, effects such as follows can be attained. That is, the body composition measuring apparatus 10 can be provided with all measurement functions for the types of body composition data desired by each member of the ordinary household (e.g., father, mother, child, grandparent, etc.).

Furthermore, according to the body composition measuring apparatus 10 and the body composition measurement system 1 according to the first embodiment of the present invention, in such a case in which a child having grown up has become seriously involved in sports and desires to have a measuring function of a particular part of a muscle, i.e., in which a measurement function of a different type of body composition data is additionally needed accompanied by the child's growth, it would not be necessary to purchase a new body composition measuring apparatus having a measurement function of such a special type of body composition data, but merely purchasing a new function adding device 301 enables the measurement of such a special type of body composition data.

Thus, the body composition measuring apparatus 10 according to the first embodiment of the present invention is highly advantageous in a case in which multiple users who use the body composition measuring apparatus 10 desire different functions, and also in a case in which a new measurement function is needed after the purchase of the body composition measuring apparatus 10.

However, in the above embodiment, description was given of an example in which the base plate unit of the body composition measuring apparatus 10 is provided with a weighing function, and in which the function adding devices are used to add functions to calculate "body fat percentage", "lean body mass", and "basic metabolic rate", but a function that can be added is not limited thereto. Furthermore, for example, the base plate unit of the body composition measuring apparatus 10 may be provided with a weighing function and a body fat percentage calculating function, and function adding devices may be used to add functions of calculating "lean body mass", "basic metabolic rate", etc. Any combination can be conceived of functions provided at the base plate unit of the body composition measuring apparatus 10 and functions that can be added using the function adding devices.

Modifications

In the following, description is given of a modification of the body composition measuring apparatus and the body composition measurement system according to the first embodiment of the present invention. To avoid repeating descriptions, description is given of differences from the body composition measuring apparatus and the body composition measurement system according to the first embodiment. The differences between the body composition measuring apparatus and the body composition measurement system according to the modification of the first embodiment and those according to the first embodiment are mainly as follows.

That is, the body composition measuring apparatus 10 according to the first embodiment is capable of calculating body composition data by executing a calculation program recorded in the function adding device 301 only in a state in which the function adding device 301 is connected to the interface 191. In contrast, according to this modification, when the function adding device 301 is connected to the interface 191, the CPU 126 of the body composition measuring apparatus 10 reads a calculation program stored in the function adding device 301, to store the calculation program to the memory storage unit 122.

In other words, the body composition measuring apparatus 10 according to this modification, when the function adding device 301 is connected to the interface 191, copies a calculation program stored in the function adding device 301 onto the memory storage unit 122. That is, in the body composition measuring apparatus 10 according to the modification, the memory storage unit 122 serves as the storage device (first storage device) 16 that stores at least one calculation program (at least one piece of first information) obtained from the function adding device 301.

As in the first embodiment, each function adding device 301 has recorded therein, together with a calculation program, "information indicating a type of body composition data" in association with the calculation program, the type of body composition data measurable by the execution of the calculation program. When the CPU 126 obtains a calculation program from the function adding device 301 for storage into the memory storage unit 122, the CPU 126 obtains "information indicating a type of body composition data" from the function adding device 301 for storage into the memory storage unit 122.

According to the present modification, the processes of Steps S2 and S6 indicated in the flowchart in Fig. 3 differ from those in the first embodiment. First, in Step S2, the CPU 126 causes the function display unit 161 to be lit for a type of body composition data indicated by the "information indicating a type of body composition data" stored in the memory storage unit 122. A user is able to easily know, viewing the display of the function display unit 161, the type of body composition data that can be measured at present without having the function adding device 301 connected to the interface 191.

Furthermore, in Step S6, in the first embodiment, the CPU 126 reads the calculation program from the function adding device 301, whereas in the modification, the CPU 126 reads the calculation program from the memory storage unit 122. That is, in the present modification, the CPU 126 reads a calculation program that is associated with the "information indicating a type of body composition data" indicating the body composition data set in Step S3, the calculation program being read from the memory storage unit 122. Thus, the CPU 126 serves as a controller that reads, from the memory storage unit 122 (first storage device), at least one calculation program (at least one piece of first information) from among at least one calculation program stored in the memory storage unit 122. The CPU 126 serving as the calculator 11, when the calculation program is supplied from the controller, calculates body composition data indicating a type of body composition corresponding to the calculation program based on the calculation program supplied from the controller, the physical characteristics information (second information), and the bioelectrical impedance Z.

In order to prevent fraudulent copying of the calculation program recorded in the function adding device 301 into multiple body composition measuring apparatuses 10, the calculation program may be provided in advance with a process of prohibiting multiple copying or a process of making the calculation program be deleted after being copied. Furthermore, in a case of permitting copying the calculation program from the function adding device 301 onto a specified number of body composition measuring apparatuses 10, the calculation program may be provided with a process of prohibiting copying of the program for a number of times exceeding a specified number.

As described in the foregoing, according to the modification of the first embodiment of the present invention, the same effects are attainable as those of the body composition measuring apparatus and the body composition measurement system according to the first embodiment.

Additionally, according to the body composition measuring apparatus and the body composition measurement system of the present modification, when the function adding device 301 is connected to the interface 191, the calculation program recorded in the function adding device 301 is read by the CPU 126 of the body composition measuring apparatus 10 and is stored in the memory storage unit 122. In other words, when the function adding device 301 is connected to the interface 191, the calculation program recorded in the function adding device 301 is copied onto the body composition measuring apparatus 10.

Therefore, for the function adding device 301 for which the copying of the calculation program on the body composition measuring apparatus 10 has already been completed, the inconvenience of connecting the function adding device 301 to the interface 191 each time to have the body composition data measured can be avoided. Furthermore, an exterior configuration of the body composition measuring apparatus 10 when measuring the body composition data is simplified.

Second Embodiment

In the following, description will be given of a body composition measuring apparatus and a body composition measurement system according to a second embodiment of the present invention. To avoid repeating descriptions, description will be given of differences from the body composition measuring apparatus and the body composition measurement system according to the first embodiment. The body composition measuring apparatus and the body composition measurement system according to the second embodiment differ from those according to the first embodiment mainly as follows.

That is, in the above-described first embodiment, it is configured so that it is not the body composition measuring apparatus 10, but the function adding device 301, that is provided with a calculation program for calculating body composition data. However, the configuration is not limited thereto, and it may be configured so that the body composition measuring apparatus 10 and the function adding device 301 work in cooperation to calculate the body composition data.

Accordingly, in the second embodiment of the present invention, multiple calculation programs respectively for calculating body composition data are stored in advance in the memory storage unit 122 (for example, ROM 122b) of the body composition measuring apparatus 10 but with restrictions on execution. Thus, the memory storage unit 122 serves as a storage device (or a second storage device) 16 having stored in advance multiple calculation programs, each for calculating the body composition data using the physical characteristics information (the second information) set and the bioelectrical impedance measured. The calculation programs bound with restrictions for execution are those programs that can be executable when the restrictions for execution are removed with a "restriction release key specifying a particular calculation program and instructing to release the restriction of the specified calculation program". The restriction release key is recorded in the function adding device 301. In the present example, one restriction release key corresponds to one calculation program.

Furthermore, the function adding device 301 has recorded therein, together with a restriction release key, "information indicating a type of body composition data" in association with the restriction release key, the type of body composition data being measurable with the restriction release key. When the body composition measuring apparatus 10 is set to the "body composition measurement mode", the CPU 126 causes the function display unit 161 to be lit, the function display unit 161 corresponding to a type of body composition data indicated by the "information indicating a type of body composition data" recorded in the function adding device 301. A user visually recognizes the display of the function display unit 161, thereby to readily determine the type of body composition data measurable at present without connecting the function adding device 301 to the interface 191.

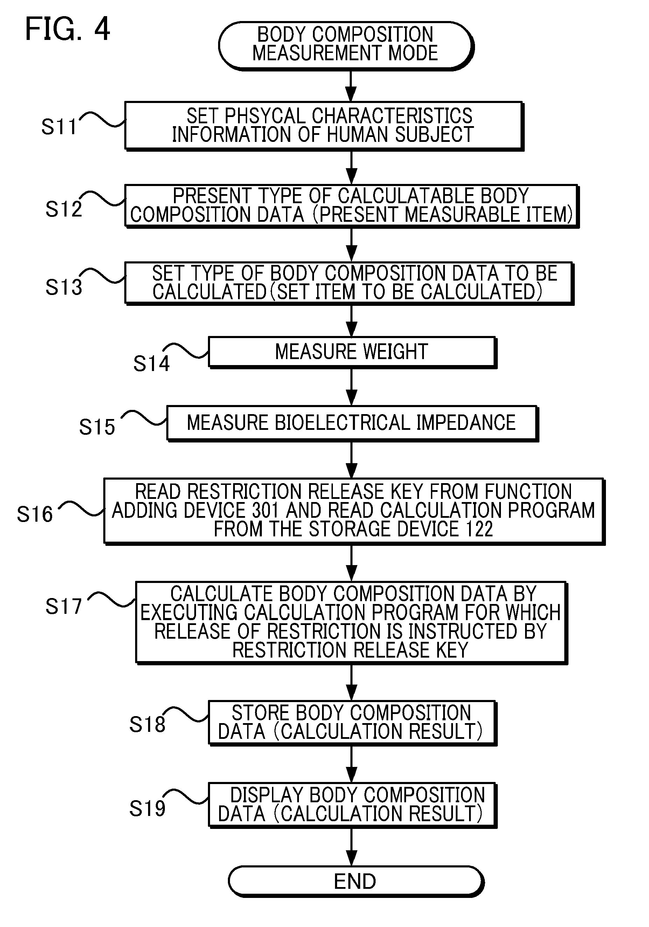

In the following, description will be given of an example of the measurement process of body composition data by the body composition measuring apparatus 10 according to the second embodiment of the present invention. Fig. 4 is a flowchart showing the measurement process of body composition data by the body composition measuring apparatus 10 according to the second embodiment of the present invention.

Steps S11 to S15 in the flowchart shown in Fig. 4 are steps for performing the same processes as Steps S1 to S5 described with reference to Fig. 3. Therefore, to avoid repeating descriptions, the processes of in and after Step S16 will be described.

When the measurement of the bioelectrical impedance Z by the voltage measurement circuit 110 is completed in Step S15, the CPU 126 reads, from the function adding device 301, a restriction release key specifying a particular calculation program and instructing to release the restriction for execution (Step S 16).

Specifically, in the present example, the CPU 126 reads, from the function adding device 301-1, a restriction release key instructing release of restriction of the calculation program for calculating "body fat percentage", reads, from the function adding device 301-2, a restriction release key instructing release of restriction of the calculation program for calculating "lean body mass ", and reads, from the function adding device 301-3, restriction release key instructing release of restriction of the calculation program for calculating "basic metabolic rate".

Then, the CPU 126, in Step S16, reads from the memory storage unit 122 the calculation program for which the release of restriction has been instructed by the read restriction release key, to release the restriction of execution thereof. The CPU 126 then executes each of the calculation programs using the physical characteristics information and bioelectrical impedance Z, thereby to calculate the body composition data (Step S17). The specific calculation process performed by executing the calculation program in Step S17 is the same as the calculation process in Step S7 of the flowchart shown in Fig. 3.

When the calculation of body composition data in Step S 17 is completed, the CPU 126 causes the calculated pieces of body composition data to be stored in the RAM 122a of the memory storage unit 122 (Step S18), to be displayed on the display unit 202 (Step S19), and ends the measurement process of the body composition data. Not only the body composition data calculated in Step S17, but also the weight calculated in Step S 14, may be displayed on the display unit 202 in Step S 19 together with the body composition data.

The calculation program stored in the ROM 122b is a program having incorporated an unlocking process for removing the restriction of execution thereof when the restriction release key is input. In a case thus configured, a restriction release key used for removing restriction of a particular calculation program may be recorded in the function adding device 301. In other words, the ROM 122b (storage device) may store therein multiple calculation programs, each for calculating a particular type of the body composition data based on the physical characteristics information and the bioelectrical impedance Z, each of the calculation programs being restricted from execution and having embedded therein an unlocking process of releasing the restriction by a restriction release key for releasing the restriction.

As described above, according to the second embodiment of the present invention, the same effects can be attained as those according the first embodiment.

Additionally, because a restriction release key is recorded in the function adding device 301, the required storage capacity of the function adding device 301 can be reduced in comparison with a case in which the calculation program is recorded therein. Furthermore, although a restriction release key is transmitted from the function adding device 301 to the body composition measuring apparatus 10, a calculation program is not transmitted. Supposedly, if a calculation program were transmitted and the calculation program were intercepted in the midst of transmission, the calculation program might be analyzed, or the calculation program might be diverted for use in a fraudulent device. However, in the second embodiment, because only the restriction release key is transmitted, the fraudulent acquisition, etc., of a calculation program that possibly takes place due to the transmission of the calculation program can be prevented.

Modifications

In the following, description will be given of a body composition measuring apparatus and a body composition measurement system according to a modification of the second embodiment of the present invention. To avoid repeating descriptions, differences from the body composition measuring apparatus and the body composition measurement system are described.

In the body composition measuring apparatus 10 according to the second embodiment, the body composition measuring apparatus 10 can remove the restriction of execution of a calculation program stored in the memory storage unit 122 by using a restriction release key stored in the function adding device 301 only in a state in which the function adding device 301 is connected to the interface 191. In contrast, in this modification, when the function adding device 301 is connected to the interface 191, the CPU 126 of the body composition measuring apparatus 10 reads a restriction release key stored in the function adding device 301, and has the restriction release key stored in the memory storage unit 122.

That is, the body composition measuring apparatus 10 according to the modification copies a restriction release key recorded in the function adding device 301 onto the memory storage unit 122 when the function adding device 301 is connected to the interface 191. Thus, the memory storage unit 122 of the body composition measuring apparatus 10 of the modification serves as the storage device (first storage device) 16 that stores at least one restriction release key (at least one piece of first information) obtained from the function adding device 301.

Like in the second embodiment, each function adding device 301 has recorded therein, together with a restriction release key, "information indicating a type of body composition data" in association with the restriction release key, the type of body composition data being measurable through the use of the restriction release key. The CPU 126, when it obtains a restriction release key from the function adding device 301 for storage into the memory storage unit 122, obtains from the function adding device 301 "information indicating a type of body composition data" for storage in the memory storage unit 122 as well.

In the present modification, the processes of Step S12 and Step S16 in the flowchart shown in Fig. 4 differ from the second embodiment. First, in Step S12, the CPU 126 causes the function display unit 161 corresponding to a type of body composition data indicated by the "information indicating a type of body composition data" stored in the memory storage unit 122 to be lit. The user visually recognizes the display of the function display unit 161, thereby easily knowing the type of body composition data that can be measured at present without connecting the function adding device 301 to the interface 191.

Furthermore, in Step S16, in the above second embodiment, a restriction release key is read from the function adding device 301, whereas in the modification, a restriction release key is read from the memory storage unit 122. That is, in the modification, the CPU 126 of the body composition measuring apparatus 10 reads at least one restriction release key from the memory storage unit 122 in Step S16, the restriction release key being stored in the memory storage unit 122. Thus, the CPU 126 serves as a controller that reads, from the memory storage unit 122 (first storage device), at least one restriction release key (at least one piece of first information) from among at least one restriction release key stored in the memory storage unit 122. The CPU 126 serving as a calculator, when the restriction release key and the calculation program are supplied from the controller, releases the restriction of the calculation program and executes the read calculation program, thereby to calculate the body composition data by using the physical characteristics information (second information) and the bioelectrical impedance Z.

In order to prevent fraudulent copying of the restriction release key recorded in the function adding device 301 into multiple body composition measuring apparatuses 10, the restriction release key may be provided in advance with a process of prohibiting multiple copying or a process of deleting the restriction release key after being copied. Furthermore, in a case of permitting copying the restriction release key from the function adding device 301 to a specified number of body composition measuring apparatuses 10, the restriction release key may be provided with a process of prohibiting copying of the key for a number of times exceeding a specified number.

As described above, according to the modification of the second embodiment of the present invention, the same effects are attainable as those of the body composition measuring apparatus and the body composition measurement system according to the second embodiment.

Additionally, according to the body composition measuring apparatus and the body composition measurement system of the present modification, when the function adding device 301 is connected to the interface 191, the restriction release key recorded in the function adding device 301 is read by the CPU 126 of the body composition measuring apparatus 10 and is stored in the memory storage unit 122. Therefore, for the function adding device 301 for which the copying of the restriction release key on the body composition measuring apparatus 10 has already been completed, the inconvenience of connecting the function adding device 301 to the interface 191 each time to have the body composition data measured can be avoided. Furthermore, an exterior configuration of the body composition measuring apparatus 10 when measuring the body composition data is simplified.

The present modification may be further modified as follows. That is, instead of having a restriction release key obtained from the function adding device 301 stored in the memory storage unit 122, an identifier indicating release of restriction may be assigned to a calculation program for which the release of restriction is instructed by the restriction release key at a time of obtaining the restriction release key from the function adding device 301. Accordingly, because the CPU 126 is able to specify a calculation program for which restriction for execution is to be removed in actually calculating the body composition data, there is no need to have the restriction release key stored in the memory storage unit 122. Instead, the CPU 126 stores, in the memory storage unit 122, an identifier indicating release of restriction in association with a calculation program for which restriction of execution is to be removed.

Specifically, the CPU 126 serving as a controller, upon obtaining a restriction release key from the function adding device 301, reads a calculation program specified by the restriction release key from the memory storage unit 122 (calculation program storage device), to store, in the memory storage unit 122, an identifier indicating release of restriction in association with the calculation program. In Step S17, the CPU 126 serving as a calculator executes a particular calculation program that is associated with the identifier, using the body characteristics information (second information) and the bioelectrical impedance, thereby to calculate body composition data.

Third Embodiment

In the following, description will be given of a body composition measuring apparatus and a body composition measurement system according to a third embodiment of the present invention. To avoid repeating descriptions, description will be given of differences from the body composition measuring apparatus and the body composition measurement system according to the first embodiment. The body composition measuring apparatus and the body composition measurement system according to the third embodiment differ from those according to the first embodiment mainly as follows.

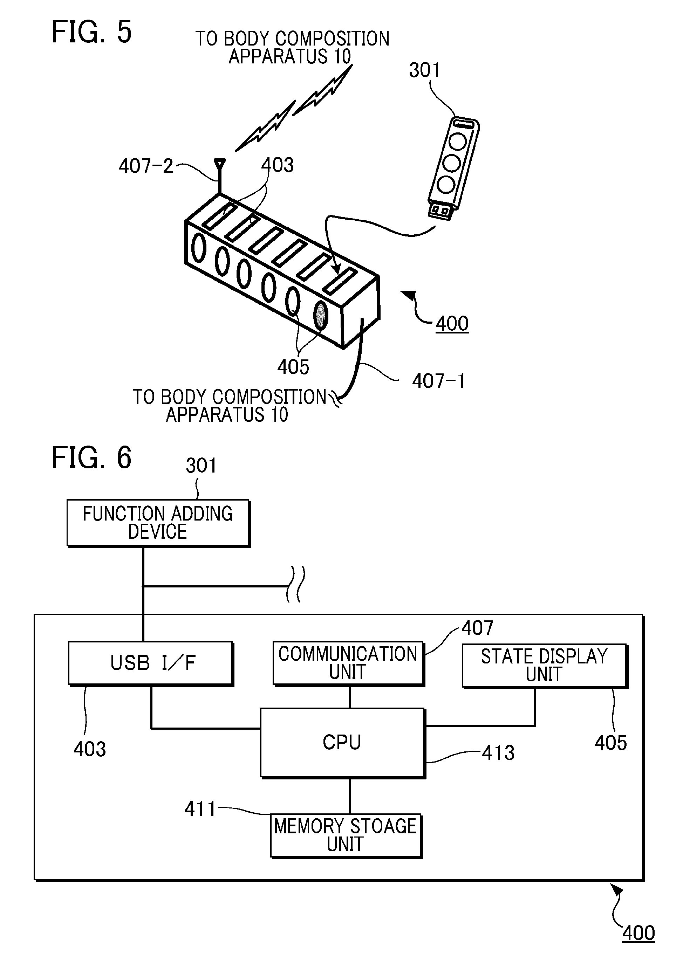

That is, in the first embodiment, the body composition measuring apparatus 10 is provided with the interface 191, and it is configured so as to connect, by means of the interface 191, the function adding device 301 directly to the body composition measuring apparatus 10. On the other hand, the body composition measurement system 1 according to the third embodiment is provided with the body composition measuring apparatus 10, the function adding device 301, and an information transmission device 400 (described later in detail with reference to Figs. 5 and 6) to which the function adding device 301 is connected and which transmits a calculation program stored in the function adding device 301 to the body composition measuring apparatus 10.

In the present embodiment, the body composition measuring apparatus 10 is provided with a communication unit (not shown) for enabling wireless or wire communication and receives a calculation program from the information transmission device 400 by means of the communication unit. Thus configured, the function adding device 301 does not have to be connected to the body composition measuring apparatus 10. Therefore, an exterior configuration of the body composition measuring apparatus 10 is additionally simplified.

The communication unit provided at the body composition measuring apparatus 10 is, for example, a communication module enabling wireless communication with the information transmission device 400. Some examples of the communication module are a communication module for connecting the body composition measuring apparatus 10 and the information transmission device 400 by a short-distance radio communication in accordance with a Bluetooth (registered trademark) standard, a communication module Wi-Fi (registered trademark)-connecting the body composition measuring apparatus 10 and the information transmission device 400 through a wireless LAN (Local Area Network), etc.

Like in the first embodiment, the function adding device 301 has recorded therein, together with a calculation program, "information indicating a type of body composition data" in association with the calculation program, the type of body composition data being measurable with the restriction release key. Therefore, the information indicating a type of body composition data is transmitted from the information transmission device 400 to the body composition measuring apparatus 10 together with a calculation program.

The CPU 126, upon receiving the calculation program and the "information indicating a type of body composition data" transmitted from the information transmission device 400 via the communication unit, causes the function display unit 161 corresponding to a type of body composition data indicated by the "information indicating a type of body composition data" to be lit. As a result, even if the information transmission device 400 is located at a remote location from the body composition measuring apparatus 10, a user simply has to view the function display unit 161, thereby easily learning of a function that can be added to the body composition measuring apparatus 10 and a function that has actually been added up to the present.

In the following, description will be given of the information transmission device 400 configured to transmit information recorded in the function adding device 301 to the body composition measuring apparatus 10. Fig. 5 is a perspective view showing an exterior configuration of the information transmission device 400 for transmitting information recorded in the function adding device 301 to the body composition measuring apparatus 10 according to the third embodiment of the present invention. Fig. 6 is a block diagram showing a system configuration example of the information transmission device 400 configured to transmit information recorded in the function adding device 301 to the body composition measuring apparatus 10 according to the third embodiment of the present invention.

As shown in Figs. 5 and 6, the information transmission device 400 is provided with interfaces 403 for connecting the respective function adding devices 301, state display units 405 being light members that are lit when the function adding device 301 is connected to the interface 403 at a corresponding position, a communication unit 407 for communicating with the communication unit of the body composition measuring apparatus 10, a memory storage unit 411 such as a RAM, etc., and a CPU 413 for controlling each unit of the information transmission device 400. In the example shown in Fig. 5, the communication unit 407 can be, for example, a communication unit 407-1 that communicates by wire with the communication unit (not shown) with the body composition measuring apparatus 10, and a communication unit 407-2 for wirelessly communicating with the communication unit of the body composition measuring apparatus 10. It is of course the case that the communication unit 407 can be either the communication unit 407-1 or the communication unit 407-2, although both of them are shown in Fig. 5.

In the body composition measurement system 1 according to the third embodiment, the function adding device 301 is connected to the information transmission device 400 provided with multiple connectors (the interfaces 403 in the present example), each for connecting to the function adding device 301 in a communicable manner and an information transmitter (the communication unit 407 and the CPU 413 in the present example) that obtains a calculation program (first information) from the function adding device 301 connected to one of the connectors and wirelessly or by wire transmits the calculation program to the body composition measuring apparatus 10. The body composition measuring apparatus 10 is provided with an information receiver that receives the calculation program transmitted by the information transmission device 400.

When the function adding device 301 is connected to the interface 403 of the information transmission device 400, the CPU 413 transmits a calculation program, etc., recorded in the function adding device 301 to the body composition measuring apparatus 10 by means of the communication unit 407. Furthermore, a user visually recognizes the ON or OFF state of the state display unit 405, thereby confirming whether the function adding device 301 has been connected to the information transmission device 400 without fail.

As described above, according to the third embodiment of the present invention, the same effects as the body composition measuring apparatus and the body composition measurement system according to the first embodiment can be attained. Furthermore, it is possible to provide the body composition measuring apparatus 10 and the body composition measurement system 1 in which an exterior configuration of the body composition measuring apparatus 10 is further simplified in comparison with that of the first embodiment. Also, the size of the body composition measuring apparatus 10 is reduced in comparison with that of the first embodiment.

For convenience of description, description was given of an example in which the third embodiment is applied to the body composition measuring apparatus 10 and the body composition measurement system 1 according to the first embodiment. However, it is of course the case that the third embodiment can be applied to the body composition measuring apparatus 10 and the body composition measurement system 1 according to any one of the modifications of the first embodiment, the second embodiment, and the modifications of the second embodiment.

Fourth Embodiment

In the following, description will be given of a body composition measuring apparatus and a body composition measurement system according to a fourth embodiment of the present invention. To avoid repeating descriptions, description will be given of differences from the body composition measuring apparatus and the body composition measurement system according to the first embodiment. The body composition measuring apparatus and the body composition measurement system according to the fourth embodiment differ from those according to the first embodiment mainly as follows.

That is, the body composition measuring apparatus 10 according to the fourth embodiment works in cooperation with an activity meter (not shown) that generates activity amount data indicating activity amount of a human subject, and the body composition data and the activity amount data are centrally managed by the body composition measuring apparatus 10. Furthermore, the latest body composition data calculated by the body composition measuring apparatus 10 is used for calculation of the activity amount data by the activity meter, thereby enhancing calculation accuracy for the activity amount data. Some examples of the "activity amount data" are "number of steps", "consumed calories", "activity intensity", "exercise", etc.

The body composition measuring apparatus 10 according to the present embodiment has a communication module that enables wireless communication with the activity meter. Some examples of the communication module are a communication module for connecting the body composition measuring apparatus 10 and the activity meter by a short-distance radio communication in accordance with a Bluetooth (registered trademark) standard, a communication module Wi-Fi (registered trademark)-connecting the body composition measuring apparatus 10 and the activity meter through a wireless LAN (Local Area Network), etc. The communication module serves as a communication unit that transmits and receives data between the body composition measuring apparatus 10 and the activity meter. The activity meter is also provided with a similar communication module as is provided at the body composition measuring apparatus 10. In a case in which the activity meter is provided with a terminal directly attachable to the interface 191 of the body composition measuring apparatus 10, the interface 191 may be used to directly connect the body composition measuring apparatus 10 and the activity meter. In other words, the body composition measuring apparatus 10 is provided with a body composition data transmitter that transmits body composition data to the activity meter that generates activity amount data indicating the activity amount of the human subject; and an activity amount data receiver that receives the activity amount data generated by the activity meter.

Thus configuring the body composition measuring apparatus 10 and the activity meter enables transmission of at least one of weight data and various types of body composition data calculated by the body composition measuring apparatus 10 to the activity meter. Transmission timing of at least one of weight data and various types of body composition data from the body composition measuring apparatus 10 to the activity meter is, for example, a timing at which the body composition measuring apparatus 10 becomes connected to the activity meter in a communicable manner.

Furthermore, at a timing after the body composition measuring apparatus 10 and the activity meter become connected with each other in a communicable manner, at least one of weight data and various types of body composition data may be transmitted from the body composition measuring apparatus 10 to the activity meter in response to a predetermined operation by a user at the body composition measuring apparatus 10 or at the activity meter.

When at least one of the weight data and the body composition data is transmitted from the body composition measuring apparatus 10 to the activity meter in the above-described process, the activity meter receives the at least one of the weight data and the body composition data, thereby to update the records on the at least one of the weight data and the body composition data for the human subject. Then, the activity meter uses at least one of the updated weight data and the updated body composition data, to calculate the activity amount of the human subject. As a result, the accuracy of the activity amount data calculated by the activity meter is enhanced.

Furthermore, the activity meter transmits the activity amount data calculated in the above-described process to the body composition measuring apparatus 10. The data can be transmitted, for example, when the activity meter is connected to the body composition measuring apparatus 10 in a communicable manner. Furthermore, after the activity meter and the body composition measuring apparatus 10 are connected in a communicable manner, the activity amount data may be transmitted from the activity meter to the body composition measuring apparatus 10 in response to a predetermined operation by a user at the activity meter or at the body composition measuring apparatus 10.

The activity amount data thus transmitted from the activity meter to the body composition measuring apparatus 10 is received by the body composition measuring apparatus 10 for storage into the memory storage unit 122 in association with the body composition data. That is, the body composition measuring apparatus 10 has a data manager that stores the body composition data and the activity amount data in association with each other. Therefore, the activity amount data and at least one of the weight data and the body composition data can be centrally managed by the body composition measuring apparatus 10.

Specifically, for example, the CPU 126 of the body composition measuring apparatus 10 graphs the activity amount data and at least one of the weight data and the body composition data under the same time axis so that the data can be compared, for display on the display unit 202. As a result, the user merely has to view the displayed graphs on the display unit 202 to recognize, at a glance, the timewise change of weight and body composition corresponding to the activity amount of the human subject.

The function adding device 301 may have recorded therein a computer program for executing various functions for centrally managing the activity amount data and at least one of the weight data and the body composition data such as a function of associating and graphing the activity amount data and at least one of the weight data and the body composition data.

As a result, even after the purchase of the body composition measuring apparatus 10, various functions, desired by a user, for centrally managing the activity amount data and at least one of the weight data and the body composition data can be added to the body composition measuring apparatus 10.

In the present embodiment, for convenience of description, the activity meter is given as an example for a measurement device that works in cooperation with the body composition measuring apparatus 10, but it is of course the case that such a measurement device is not limited to the activity meter. For example, a measurement device such as a sleep meter, a pedometer, etc., may work in coordination with the body composition measuring apparatus 10. Data collected by such a measurement device and the data collected by the body composition measuring apparatus 10 can be centrally managed.

As described above, according to the fourth embodiment of the present invention, the same effects can be attained as those of the body composition measuring apparatus and the body composition measurement system according to the first embodiment. Furthermore, it is possible to provide a body composition measuring apparatus and a body composition measurement system in which the weight data and the body composition data for a human subject and data collected by another measurement apparatus for the same human subject are centrally managed.

Specifically, for example, the weight data and the body composition data can be associated with various data collected by another measurement device, as a visual representation to a human subject. Therefore, the human subject merely has to view a graph such as described above, etc., displayed on the display unit 202, thereby to easily understand how the weight data and the body composition data and various types of data change from time to time, and to recognize the correlations between multiple data easily.

Furthermore, because data calculated by another measurement device has reflected the latest weight data and body composition data calculated by the body composition measuring apparatus 10, the accuracy of the data calculated by another measurement device is enhanced.

It is of course the case that the fourth embodiment can be applied to any one of the above-described first to the third embodiments and the modifications.

In the above, the present invention has been described according to the first to the fourth embodiments and the modifications, but the present invention is not limited to the above examples. It is of course the case that modifications and applications to and of the present invention can be made within the spirit of the present invention. In the following, description is given of application examples. To avoid repeating descriptions, only the differences from the first to the fourth embodiments and the modifications are described.

First Application Example

A communication terminal device, such as a device called a smartphone, can be used instead of using the function adding device 301. In other words, a recording unit provided at a communication terminal device may be used as the function adding device 301 (i.e., a recording medium is provided in a communication terminal device that performs wireless or wire communication). Therefore, the body composition measuring apparatus 10 according to the first application example obtains a calculation program (first information) or a restriction release key (first information) from a communication terminal device such as called a smartphone. In the following, description will be given of the body composition measuring apparatus 10 and the body composition measurement system 1 according to the present application example.

The body composition measuring apparatus 10 is provided with a communication module for wireless or wire communicating with the communication terminal device. Some examples of radio communication modules are a communication module for connecting the body composition measuring apparatus 10 and the communication terminal device by a short-distance radio communication in accordance with a Bluetooth (registered trademark) standard, a communication module Wi-Fi (registered trademark)-connecting the body composition measuring apparatus 10 and the communication terminal device through a wireless LAN (Local Area Network), etc.

The communication terminal device downloads a calculation program or a restriction release key as an application software called an "app", for transmission to the body composition measuring apparatus 10. The body composition measuring apparatus 10 receives and obtains the calculation program or the restriction release key transmitted from the communication terminal device via the communication module.

Thus configured, a user only has to download a calculation program or a restriction release key for a desired measurement function by a communication terminal device such as a smartphone, and operate the smartphone to transmit the calculation program or the restriction release key to the body composition measuring apparatus 10, thereby to add a desired measurement function to the body composition measuring apparatus 10. That is, the body composition measuring apparatus 10 can receive a calculation program or a restriction release key from a communication terminal device such as a smartphone.

As described above, according to the first application example, the same effects can be attained as the body composition measuring apparatus 10 and the body composition measurement system 1 according to each embodiment. Furthermore, it is possible to provide the body composition measuring apparatus 10 and the body composition measurement system 1 capable of adding a measurement function by using a communication terminal device such as a smartphone. That is, according to the body composition measuring apparatus 10 and the body composition measurement system 1 according to the first application example, a user uses a communication terminal device such as a smartphone and downloads a calculation program or a restriction release key, for transfer from the smartphone to the body composition measuring apparatus 10, thereby to add a measurement function to the body composition measuring apparatus 10.

Therefore, according to the body composition measuring apparatus 10 and the body composition measurement system 1 of the first application example, a user does not have to actually visit a shop, etc., where the function adding device 301 can be bought, but can easily obtain, for example, at home, etc., a calculation program or restriction release key for a desired measurement function, to add the measurement function to the body composition measuring apparatus 10.

## Second Application Example

In the above example, the body composition measuring apparatus 10 has added to it a measurement function, but a function other than a measurement function (for example, a game function or a music playing function, etc.) may be added to the body composition measuring apparatus 10. When thus configured, computer programs for executing various functions, instead of a calculation program for calculating body composition data, may be added to the body composition measuring apparatus 10. Furthermore, the body composition measuring apparatus 10 may be configured to be made to work in cooperation with a pedometer or a sleep meter, so as to manage health data of a human subject in cooperation with these measurement devices. When thus configured, a computer program for executing a process of having the body composition measuring apparatus 10 works in cooperation with a pedometer or a sleep meter is added to the body composition measuring apparatus 10.

As described in the foregoing, according to the second application example, the same effects can be attained as those of the body composition measuring apparatus 10 and the body composition measurement system 1 according to each embodiment. Furthermore, it is possible to provide the body composition measuring apparatus 10 and the body composition measurement system 1 in which not only measurement functions, but also various other functions, can be added.

## Third Application Example

In the above example, values of body composition data, being measurement results in the body composition measurement mode, are displayed on the display unit 202 in Step S9 or Step S 19. A means for displaying the measurement results is not limited to the display unit 202 of the body composition measuring apparatus 10. For example, the body composition measuring apparatus 10 may be provided with a communication function with a communication terminal device such as called a smartphone, thereby to transmit body composition data to the communication terminal device, so that body composition data being measurement results are displayed on the smartphone. Furthermore, a smartphone may be provided with a function corresponding to at least one of the operation unit 120, the state display unit 151, and the function display unit 161 of the body composition measuring apparatus 10. In this case, the body composition measuring apparatus 10 does not necessarily have to be provided with the operation unit 120, the state display unit 151, or the function display unit 161.

In the body composition measurement system 1 according to the third embodiment, the information transmission device 400 may be provided with a display unit such as an LCD, etc., so that measurement results can be displayed and viewed at the information transmission device 400, the measurement results being transmitted from the body composition measuring apparatus 10 to the information transmission device 400.

As described in the foregoing, according to the third application example, the same effects can be attained as those of the body composition measuring apparatus 10 and the body composition measurement system 1 according to each embodiment. Furthermore, it is possible to provide the body composition measuring apparatus 10 and the body composition measurement system 1 in which usability is enhanced in viewing measurement results. Furthermore, because various types of functions provided with the base plate unit of the body composition measuring apparatus 10 are allocated instead to the information transmission device 400, the base plate unit of the body composition measuring apparatus 10 can be configured as one that is simple and has excellent designed properties.

Furthermore, the above-described embodiments encompass various phases of the invention, and therefore, various aspects of the invention can be derived by appropriate combination of multiple configuration features embodied above. For example, the aspect of the invention can be derived in which some of the configuration features described above are omitted, as long as the same effects as described above can be attained.

## Description of Reference Numerals

1...body composition measurement system, 10...body composition measuring apparatus, 102... platform, 104... electrode unit, 104a, 104b... current supplying electrode, 104c, 104d... voltage measuring electrode, 108... high frequency constant current circuit, 110... voltage measurement circuit, 112... weight sensor, 114... amplifier circuit, 116... switch, 118... converter, 120... operation unit, 122... memory storage unit, 126...CPU, 151...state display unit, 161... function display unit, 191...interface, 202...display unit, 301,301-1,301-2,301-3...function adding device, 400... information transmission device, 403... interface, 405... state display unit, 407,407-1,407-2...communication unit, 411...memory storage unit.

**Claims**

1. A body composition measuring apparatus for calculating at least one type of body composition data, the body composition measuring device comprising:

   an information obtainer that obtains at least one piece of first information from each of at least one recording medium having recorded thereon the at least one piece of the first information necessary for calculating body composition data indicating a particular type of body composition;
   an information setting unit that sets second information indicating physical characteristics of a human subject;
   a measurer that measures bioelectrical impedance of the human subject; and
   a calculator that calculates body composition data indicating a type of body composition corresponding to each of the at least one piece of the first information based on the at least one piece of the first information obtained by the information obtainer, the second information, and the bioelectrical impedance.

2. The body composition measuring apparatus of Claim 1,
   wherein the first information is a calculation program for calculating the body composition data by using the second information and the bioelectrical impedance, and
   wherein the calculator, when the calculation program is obtained by the information obtainer, executes the calculation program, thereby calculating the body composition data by using the second information and the bioelectrical impedance.

3. The body composition measuring apparatus of Claim 1, further comprising a storage device having stored in advance multiple calculation programs, each for calculating the body composition data by using the second information and the bioelectrical impedance,
   wherein each of the multiple calculation programs is restricted from execution;
   wherein the first information is a restriction release key specifying a particular calculation program and instructing to release the restriction of the specified calculation program; and
   wherein the calculator reads, from the storage device, the calculation program for which the restriction has been instructed to be released by the first information and releases the restriction of the calculation program, and executes the read calculation program by using the second information and the bioelectrical impedance, thereby calculating the body composition data.

4. A body composition measuring apparatus for calculating at least one type of body composition data, the body composition measuring device comprising:

   an information obtainer that obtains at least one piece of first information from each of at least one recording medium having recorded thereon at least one piece of the first information necessary for calculating body composition data indicating a particular type of body composition;
   an information setting unit that sets second information indicating physical characteristics of a human subject;
   a measurer that measures bioelectrical impedance of the human subject;
   a first storage device that stores the at least one piece of first information obtained from each recording medium by the information obtainer;
   a calculator that calculates body composition data indicating a type of body composition corresponding to each of the at least one piece of the first information based on the at least one piece of the first information, the second information, and the bioelectrical impedance; and
   a controller that reads, from the first storage device, at least one piece of first information from among the at least one piece of first information stored in the first storage device, for supply to the calculator.

5. The body composition measuring apparatus of Claim 4,
   wherein the first information is a calculation program for calculating the body composition data by using the second information and the bioelectrical impedance; and
   wherein the calculator, when the calculation program is supplied by the controller, executes the calculation program, to calculate the body composition data by using the second information and the bioelectrical impedance.

6. The body composition measuring apparatus of Claim 4, further comprising a second storage device having stored in advance multiple calculation programs, each for calculating the body composition data by using the second information and the bioelectrical impedance,
   wherein each of the multiple calculation programs is restricted from execution;

wherein the first information is a restriction release key specifying a particular calculation program and instructing to release the restriction of the specified calculation program;

wherein the controller supplies the calculator with the restriction release key, and reads, from the second storage device, the particular calculation program for supply to the calculator, the particular calculation program being specified by the restriction release key; and

wherein the calculator, when the restriction release key and the particular calculation program are supplied by the controller, releases the restriction of the particular calculation program and executes the read calculation program, thereby to calculate the body composition data by using the second information and the bioelectrical impedance.

7. A body composition measuring apparatus comprising:

a calculation program storage device that stores therein multiple calculation programs, each for calculating body composition data indicating body composition of a human subject, with each of the multiple calculation programs being restricted from execution;

an information obtainer that obtains at least one piece of first information from each of at least one recording medium, the first information being a restriction release key specifying a particular calculation program and instructing to release the restriction of the particular calculation program;

an information setting unit that sets second information indicating physical characteristics of the human subject;

a measurer that measures bioelectrical impedance of the human subject;

a controller that reads, from the calculation program storage device, the particular calculation program specified by the first information obtained by the information obtainer, and stores an identifier in association with the calculation program in the calculation program storage device, the identifier indicating that the restriction is to be released; and

a calculator that executes the calculation program associated with the identifier, thereby to calculate the body composition data by using the second information and the bioelectrical impedance.

8. The body composition measuring apparatus of any one of Claims 1 to 7, further comprising a notifier that notifies the human subject of a type of body composition data corresponding to the at least one piece of first information when the body composition measuring apparatus changes to a state in which the at least one piece of first information can be obtained from the at least one recording medium.

9. The body composition measuring apparatus of any one of Claims 1 to 8, further comprising multiple connectors, each connecting one of the at least one recording medium to the body composition measuring apparatus in a communicable manner.

10. The body composition measuring apparatus of any one of Claims 1 to 9,
wherein the recording medium can be connected to an information transmission device having and multiple connectors, each connecting one of the at least one recording medium to the information transmission device in a communicable manner, and an information transmitter that obtains the at least one piece of first information from each of the at least one recording medium connected to one of the multiple connectors and transmits wirelessly or by wire the obtained at least one piece of first information to the body composition apparatus,
the body composition measuring apparatus further comprising an information receiver that receives the at least one piece of first information transmitted by the information transmission device.

11. The body composition measuring apparatus of any one of Claims 1 to 10,
wherein the recording medium is provided in a communication terminal device that performs wireless or wire communication, and
wherein the information obtainer receives the at least one piece of first information from the communication terminal device.

12. The body composition measuring apparatus of any one of Claims 1 to 11, further comprising:

a body composition data transmitter that transmits the body composition data to an activity meter that generates activity amount data indicating the amount of activity of the human subject;

an activity amount data receiver that receives the activity amount data generated by the activity meter; and

a data manager that stores the body composition data and the activity amount data in association with each other.

13. A body composition measuring apparatus for calculating body composition data indicating body composition of a human subject, comprising:

an information setting unit that sets second information indicating physical characteristics of the human subject;
a measurer that measures bioelectrical impedance of the human subject;
a storage device that stores therein multiple calculation programs, each for calculating a particular type of the body composition data based on the physical characteristics information and the bioelectrical impedance, each of the calculation programs being restricted from execution and having embedded therein an unlocking process of releasing the restriction by a restriction release key for releasing the restriction;
a restriction release key obtainer that obtains the restriction release key from a recording medium in which the restriction release key for at least one of the multiple calculation programs is stored; and
a calculator that executes the calculation program for which the restriction has been released by the restriction release key, thereby to calculate the body composition data based on the physical characteristics information and the bioelectrical impedance.

**14.** A body composition measurement system comprising:

the body composition measuring apparatus of any one of Claims 1 to 13; and
a recording medium having recorded thereon the first information being information necessary for calculation of body composition data.

# FIG. 1

MEASURABLE
ITEMS

BODY FAT %

LEAN BODY MASS

BASIC METABOLIC RATE

# FIG. 2A

**1**

| 301-1 | 301-2 | 301-3 |
|-------|-------|-------|
| FUNCTION ADDING DEVICE | FUNCTION ADDING DEVICE | FUNCTION ADDING DEVICE |

BODY COMPOSITION APPARATUS

USB I/F

191

104 — ELECTRODE UNit    104c,104d    104a,104b

VOLTAGE MEASURING ELECTRODE

CURRENT SUPPLYING ELECTRODE

112

WEIGHT SENSOR

AMPLIFIER CIRCUIT

114

VOLTAGE MEASUREMENT CIRCUIT

110

HIGH FREQUENCY CONSTANT CURRENT CIRCUIT

108

116 — SWITCH

118 — A/D CONVERTER

120

OPERATION UNIT

MEMORY STORAGE UNIT
- RAM
- ROM
- REWRITABLE MEMORY

122a
122b  122
122c

CPU

12
6

DISPLAY UNIT

202

FUNCTION DISPLAY UNIT

161

STATE DISPLAY UNIT

151

FIG. 2B

FIG. 3

```
        ┌─────────────────────────┐
        │   BODY COMPOSITION      │
        │   MEASUREMENT MODE      │
        └─────────────────────────┘
                    │
                    ▼
S1  ┌──────────────────────────────────────────┐
    │ SET PHSYCAL CHARACTERISTICS INFORAMTION  │
    │          OF HUMAN SUBJECT                │
    └──────────────────────────────────────────┘
                    │
                    ▼
S2  ┌──────────────────────────────────────────────┐
    │      PRESENT TYPE OF CALCULATABLE BODY       │
    │ COMPOSITION DATA (PRESENT MEASURABLE ITEM)   │
    └──────────────────────────────────────────────┘
                    │
                    ▼
S3  ┌──────────────────────────────────────────────┐
    │   SET TYPE OF BODY COMPOSITION DATA TO BE     │
    │  CALCULATED (SET ITEM TO BE CALCULATED)       │
    └──────────────────────────────────────────────┘
                    │
                    ▼
S4      ┌──────────────────────────────┐
        │      MEASURE WEIGHT          │
        └──────────────────────────────┘
                    │
                    ▼
S5  ┌──────────────────────────────────────────┐
    │   MEASURE BIOELECTRICAL IMPEDANCE        │
    └──────────────────────────────────────────┘
                    │
                    ▼
S6  ┌──────────────────────────────────────────┐
    │  READ CALCULATION PROGRAM FROM           │
    │     FUNCTION ADDING DEVICE 301           │
    └──────────────────────────────────────────┘
                    │
                    ▼
S7  ┌──────────────────────────────────────────┐
    │  CALCULATE BODY COMPOSITION              │
    │  DATA BY EXECUTING READ                  │
    │  CALCULATION PROGRAM                     │
    └──────────────────────────────────────────┘
                    │
                    ▼
S8  ┌──────────────────────────────────────────┐
    │     STORE BODY COMPOSITION               │
    │  DATA (CALCULATION RESULT)               │
    └──────────────────────────────────────────┘
                    │
                    ▼
S9  ┌──────────────────────────────────────────┐
    │   DISPLAY BODY COMPOSITION               │
    │  DATA (CALCULATION RESULT)               │
    └──────────────────────────────────────────┘
                    │
                    ▼
        ┌──────────────────────────┐
        │          END             │
        └──────────────────────────┘
```

FIG. 4

```
        ┌─────────────────────────┐
        │   BODY COMPOSITION      │
        │   MEASUREMENT MODE      │
        └─────────────────────────┘
                    │
                    ▼
S11  ┌─────────────────────────────────────────┐
     │   SET PHSYCAL CHARACTERISTICS           │
     │   INFORMATION OF HUMAN SUBJECT          │
     └─────────────────────────────────────────┘
                    │
                    ▼
S12  ┌─────────────────────────────────────────┐
     │   PRESENT TYPE OF CALCULATABLE BODY     │
     │   COMPOSITION DATA (PRESENT MEASURABLE  │
     │   ITEM)                                 │
     └─────────────────────────────────────────┘
                    │
                    ▼
S13  ┌─────────────────────────────────────────┐
     │   SET TYPE OF BODY COMPOSITION DATA TO  │
     │   BE CALCULATED (SET ITEM TO BE         │
     │   CALCULATED)                           │
     └─────────────────────────────────────────┘
                    │
                    ▼
S14  ┌─────────────────────────────────────────┐
     │          MEASURE WEIGHT                 │
     └─────────────────────────────────────────┘
                    │
                    ▼
S15  ┌─────────────────────────────────────────┐
     │   MEASURE BIOELECTRICAL IMPEDANCE       │
     └─────────────────────────────────────────┘
                    │
                    ▼
S16  ┌─────────────────────────────────────────┐
     │   READ RESTRICTION RELEASE KEY FROM     │
     │   FUNCTION ADDING DEVICE 301 AND READ   │
     │   CALCULATION PROGRAM FROM THE STORAGE  │
     │   DEVICE 122                            │
     └─────────────────────────────────────────┘
                    │
                    ▼
S17  ┌─────────────────────────────────────────┐
     │   CALCULATE BODY COMPOSITION DATA BY    │
     │   EXECUTING CALCULATION PROGRAM FOR     │
     │   WHICH RELEASE OF RESTRICTION IS       │
     │   INSTRUCTED BY RESTRICTION RELEASE KEY │
     └─────────────────────────────────────────┘
                    │
                    ▼
S18  ┌─────────────────────────────────────────┐
     │   STORE BODY COMPOSITION                │
     │   DATA (CALCULATION RESULT)             │
     └─────────────────────────────────────────┘
                    │
                    ▼
S19  ┌─────────────────────────────────────────┐
     │   DISPLAY BODY COMPOSITION              │
     │   DATA (CALCULATION RESULT)             │
     └─────────────────────────────────────────┘
                    │
                    ▼
        ┌─────────────────────────┐
        │          END            │
        └─────────────────────────┘
```

# FIG. 5

TO BODY COMPOSITION
APPARATUS 10

301

407-2  403

400

405

TO BODY COMPOSITION
APPARATUS 10

407-1

# FIG. 6

301

| FUNCTION ADDING DEVICE |

407

| USB I/F | | COMMUNICATION UNIT | | STATE DISPLAY UNIT |

| CPU |

403

413

405

411

| MEMORY STOAGE UNIT |

400

Europäisches
Patentamt

European
Patent Office

Office européen
des brevets

# EUROPEAN SEARCH REPORT

Application Number

EP 14 17 1730

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/150916 A2 (SECA AG [CH]; KLUECKMANN KRISTIN [DE]; HOEFLER MARTIN [DE]; MUELLER MA) 8 December 2011 (2011-12-08) * abstract; claims 1,6,10; figure 7 * * page 2, line 6 - line 22 * * page 3, line 1 - page 3, line 15 * * page 4, line 18 - page 5, line 5 * * page 5, line 23 - page 5, line 28 * * page 8, line 6 - page 8, line 18 * ----- | 1-14 | INV. G01G19/50 A61B5/053 |

TECHNICAL FIELDS
SEARCHED      (IPC)

G01G
A61B

The present search report has been drawn up for all claims

| Place of search | Date of completion of the search | Examiner |
|---|---|---|
| The Hague | 24 October 2014 | Katerbau, Ragnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 14 17 1730

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2014

| Patent document cited in search report | Publication date | Patent family member(s) | Publication date |
|---|---|---|---|
| WO 2011150916 A2 | 08-12-2011 | CN 103298395 A | 11-09-2013 |
| | | DE 102011102854 A1 | 28-06-2012 |
| | | EP 2575601 A2 | 10-04-2013 |
| | | JP 2013534837 A | 09-09-2013 |
| | | US 2013131462 A1 | 23-05-2013 |
| | | WO 2011150916 A2 | 08-12-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007117525 A